# EUROPEAN PATENT APPLICATION

(11) **EP 4 697 226 A2**
(43) Date of publication of application: **18.02.2026**
(21) Application number: 25221133.9
(22) Date of filing: 19.12.2022
(51) Int. Cl.: G06F 30/20

(54) **ENVIRONMENTAL ATTRIBUTES FOR MATERIALS**

(30) Priority: 21.12.2021 EP 21216268; 21.12.2021 EP 21216269; 21.12.2021 EP 21216270; 21.12.2021 EP 21216271; 21.12.2021 EP 21216286; 21.12.2021 EP 21216292; 21.12.2021 EP 21216326; 21.12.2021 EP 21216327; 21.12.2021 EP 21216333; 04.04.2022 EP 22166573; 12.04.2022 EP 22167945; 10.05.2022 EP 22172609; 10.05.2022 EP 22172611; 10.05.2022 EP 22172615; 10.05.2022 EP 22172617; 10.05.2022 EP 22172619; 09.09.2022 EP 22194793; 09.09.2022 EP 22194800; 09.09.2022 EP 22194808; 09.09.2022 EP 22194815; 09.09.2022 EP 22194818; 14.10.2022 EP 22201672; 14.10.2022 US 202263416091 P; 18.10.2022 EP 22202183; 05.12.2022 EP 22211421
(62) Divisional of application: 22836235.6
(71) Applicant: BASF SE, 67056 Ludwigshafen am Rhein (DE)
(72) Inventor: GRUMBRECHT, Bastian, 67061 Ludwigshafen am Rhein (DE); KLOSTERHALFEN, Steffen Thomas, Stockport, SK1 3GG (GB); KRUEGER, Christian, 67056 Ludwigshafen am Rhein (DE); ALBA PEREZ, Ana, 67056 Ludwigshafen am Rhein (DE); ANDERLOHR, Christopher Alec, 67056 Ludwigshafen am Rhein (DE); BINDER, Martin, 67056 Ludwigshafen am Rhein (DE); PISTILLO, Alessandro, 67056 Ludwigshafen am Rhein (DE); WENZL, Kurt, 67117 Limburgerhof (DE)
(74) Representative: BASF IP Association

(57) **Abstract**

Disclosed are systems for producing an output material associated with a digital asset, methods for producing an output material associated with a digital asset, apparatuses for generating a digital asset, computer-implemented methods for generating a chemical passport, computer program elements for generating a digital asset, uses of an output material associated with a digital asset, uses of a digital asset, products produced from the output material and associated with a digital asset, a digital asset including one or more decentral identifier(s) and data related to the environmental impact data, apparatuses for producing a product associated with the digital asset and methods for producing a product associated with the digital asset.

## Description

### TECHNICAL FIELD

The present disclosure relates to systems for producing an output material associated with an output material passport or digital asset, methods for producing an output material associated with an output material passport or digital asset, apparatuses for generating an output material passport or digital asset, computer-implemented methods for generating a chemical passport or digital asset, computer program elements for generating an output material passport or digital asset, uses of an output material associated with an output material passport or digital asset, uses of an output material passport or digital asset, products produced from the output material and associated with an output material passport or digital asset, an output material, an output material passport or digital asset including one or more decentral identifier(s) and data related to environmental impact data, apparatuses for producing a product associated with the output material passport or digital asset and methods for producing a product associated with the output material passport or digital asset.

### TECHNICAL BACKGROUND

In supply chains the environmental impact of each supply chain participants is of great interest. Specifically in the field of chemistry, output materials are employed for a wide range of applications and are supplied to diverse value chains. In such complex systems transparency between value chain participants is hard to achieve.

### SUMMARY OF THE INVENTION

In one aspect disclosed is a system for producing an output material associated with an output material passport or a digital asset, the system comprising:
- a chemical production network configured to produce the output material, wherein the output material is produced from one or more input material(s) through one or more chemical process(s) of the chemical production network, wherein the one or more input material(s) and/or the one or more chemical process(s) are associated with environmental attribute(s);
- a production operating apparatus configured to generate the output material passport or digital asset by
   providing a decentral identifier associated with the produced output material and the one or more environmental attribute(s) of the one or more input material(s) and/or the one or more chemical process(s);
   linking the decentral identifier and the environmental attribute(s);
- optionally the chemical production network or system configured to provide the produced output material in association with the output material passport or digital asset.

In one aspect disclosed is a system for producing an output material associated with an output material passport or a digital asset, the system comprising:
- a chemical production network configured to produce the output material, wherein the output material is produced from one or more input material(s) through one or more chemical process(s) of the chemical production network, wherein the one or more input material(s) and/or the one or more chemical process(s) are associated with environmental attribute(s);
- a production operating apparatus configured to generate the output material passport or digital asset by providing a decentral identifier associated with the produced output material and linking the decentral identifier to the environmental attribute(s) of the one or more input material(s) and/or the one or more chemical process(s).

In another aspect disclosed is a system for producing an output material associated with an output material passport or digital asset, the system comprising:
- a chemical production network configured to produce the output material from one or more input material(s) through chemical process(s), wherein the one or more input material(s) and/or the chemical process(s) are associated with environmental attribute(s);
- a production operating apparatus configured to generate the output material passport or digital asset by providing and/or linking a decentral identifier associated with the output material and one or more environmental attribute(s) associated with the one or more input material(s) and/or the chemical process(s).

In another aspect disclosed is a method for producing an output material associated with an output material passport or digital asset, wherein the method comprises:
- producing the output material from one or more input material(s) through one or more chemical process(s) of a chemical production network, wherein the one or more input material(s) and/or the one or more chemical process(s) are associated with environmental attribute(s);
- generating the output material passport or digital asset by
   providing a decentral identifier associated with the produced output material and one or more environmental attribute(s) associated with the one or more input material(s) and/or the one or more chemical process(s);
   linking the decentral identifier and the one or more environmental attribute(s);
- providing the produced output material in association with the output material passport or digital asset.

In another aspect disclosed is a method for producing an output material associated with an output material passport or a digital asset, wherein the method comprises:
- producing the output material from one or more input material(s) through one or more chemical process(s) of a chemical production network, wherein the one or more input material(s) and/or the one or more chemical process(s) are associated with environmental attribute(s),
- generating the output material passport or digital asset by providing and/or linking a decentral identifier associated with the output material and one or more environmental attribute(s) of the one or more input material(s) and/or the chemical process(s).

In another aspect disclosed is an apparatus for generating a passport or digital asset associated with an output material, wherein the output material is produced from one or more input material(s) through one or more chemical process(s) of a chemical production network, wherein the one or more input material(s) and/or the one or more chemical process(s) are associated with environmental attribute(s), the apparatus comprising:
- a decentral identity provider configured to provide a decentral identifier associated with the produced output material and one or more environmental attribute(s) of the one or more input material(s) and/or the one or more chemical process(s) used to produce the output material;
- an assignor configured the decentral identifier and the environmental attribute(s);
- a passport provider configured to provide the output material passport or digital asset in association with the produced output material e.g. to a decentral network, wherein the environmental attribute(s) associated with the output material is made accessible to a consumer or user of the output material through the output material passport or digital asset.

In another aspect disclosed is a method, e.g. a computer-implemented method, for generating an output material passport or digital asset associated with an output material, wherein the output material is produced from one or more input material(s) through one or more chemical process(s) of a chemical production network, wherein the one or more input material(s) and/or the one or more chemical process(s) are associated with environmental attribute(s), the method comprising:
- providing a decentral identifier associated with the produced output material and one or more environmental attribute(s) of the one or more input material(s) and/or the one or more chemical process(s) used to produce the output material;
- linking the decentral identifier and the environmental attribute(s);
- providing the output material passport or digital asset in association with the produced output material e.g. to a decentral network, wherein the environmental attribute(s) associated with the output material is made accessible to a user of the output material through the output material passport or digital asset.

In another aspect disclosed is a computer element, such as a computer readable storage medium, a computer program or a computer program product, comprising instructions, which when executed by a computing node or a computing system, direct the computing node or computing system to carry out the steps of the computer-implemented methods disclosed herein.

In another aspect disclosed is a computer element, such as a computer readable storage medium, a computer program or a computer program product, comprising instructions, which when executed by the apparatuses disclosed herein, direct the apparatuses to carry out steps the apparatuses disclosed herein are configured to execute.

In another aspect disclosed is an output material associated with an output material passport or a digital asset as produced according to the methods disclosed herein. In another aspect disclosed is an output material associated with an output material passport or digital asset as produced according to the systems disclosed herein.

In another aspect disclosed is an output material associated with an output material passport or digital asset, wherein the output material is produced from one or more input material(s) through one or more chemical process(s) of a chemical production network, wherein the one or more input material(s) and/or the one or more chemical process(s) are associated with environmental attribute(s), wherein the output material passport or digital asset includes a decentral identifier associated with the produced output material and a link to environmental attribute(s) associated with one or more environmental attribute(s) of one or more input material(s) and/or one or more chemical process(s) used to produce the output material.

In another aspect disclosed is an output material passport or digital asset as generated according to the methods disclosed herein. In another aspect disclosed is an output material passport or digital asset as generated according to the apparatuses disclosed herein.

In another aspect disclosed is a production system for producing a product from the output material associated with the output material passport or digital asset as provided according to the systems, apparatuses or methods disclosed herein. In another aspect disclosed is a production method for producing a product from the output material associated with the output material passport or digital asset as provided according to the systems, apparatuses or methods disclosed herein.

In another aspect disclosed is a use of the output material associated with the output material passport or digital asset as disclosed herein for producing a product from the output material associated with the output material passport or digital asset.

In another aspect disclosed is a use of the output material passport or digital asset as disclosed herein for generating a product passport or digital asset associated with a product produced from the output material associated with the output material passport or digital asset. In another aspect disclosed is a method for using the digital asset generated according to the methods disclosed herein in the production of a product produced from the output material associated with the output material passport or digital asset.

In another aspect disclosed is an output material associated with a digital asset including a decentral identifier associated with the output material and linked to one or more environmental attribute(s) of the one or more input material(s) and/or the one or more chemical process(s) used to produce the output material.

In another aspect disclosed is a use of the output material associated with the output material passport or digital asset for producing a product from the output material and associating the output material passport or digital asset with the product produced from the output material. In another aspect disclosed is a use of the output material associated with the output material passport or digital asset for producing a product from the output material and deriving a product passport or digital asset from the output material passport or digital asset. In another aspect disclosed is a method for using the output material associated with the digital asset for producing a product from the output material as disclosed herein and deriving a digital asset associated with the product from the output material passport or output material digital asset.

In another aspect disclosed is a use of the output material passport or digital asset as disclosed herein for generating a product passport or digital asset associated with a product produced from the output material associated with the output material passport or digital asset.

Any disclosure and embodiments described herein relate to the methods, the systems, output materials, output material passports, digital assets and the computer elements lined out above and below. Advantageously, the benefits provided by any of the embodiments and examples equally apply to all other embodiments and examples.

### EMBODIMENTS

In the following, embodiments of the present disclosure will be outlined by ways of embodiments and/or example. It is to be understood that the present disclosure is not limited to said embodiments and/or examples.

Determining, generating includes initiating or causing to determine, generate. Providing includes "initiating or causing to access, determine, generate, send or receive". "Initiating or causing to perform an action" includes any processing signal that triggers a computing node to perform the respective action.

The methods, the systems, output materials, output material passports or digital assets and the computer elements disclosed herein provide an efficient, secure and robust way for sharing or exchanging environmental impact data across different participant nodes in value chains. In particular, by providing output material specific data via the output material passport or digital asset, environmental impacts can be shared and made transparent from the material to the product produced from such material. The output material passport or digital asset enables secure data exchange, since data access can be controlled by the material provider, e.g. the entity providing the output material. The exchanged data assets can be specific to the output material as produced and tailored to the needs of the consumer of the output material. This way an improved tracking and tracing of materials can be achieved by securely providing environmental impact data in diverse and highly complex value chains. The environmental impact of materials can hence be tracked leading to simpler, more efficient and sustainable handling of materials by value chain participants.

The output material may be obtained from one or more chemical reaction(s). The output material may refer to any type of substance or material directly or indirectly used to produce discrete products. The terms chemical product, material or chemical may be used interchangeably. The output material may include a starting material such as an educt for the chemical reaction. The output material may include an intermediate material such as an educt for an upstream chemical reaction following a downstream chemical reaction. The output material may include a chemical end product such as input material used to produce a discrete product or input material used for discrete manufacturing. The chemical end product may signify material that is used to produce discrete products.

The output material may include synthetic or natural substances. Output materials obtained from chemical reactions may include any inorganic or organic chemical substance obtained by reacting inorganic and/or organic chemical reactants. The inorganic and organic chemical reactants may be naturally occurring or can be obtained from chemical reactions. Chemical reactions may include any chemical reaction commonly known in the state of the art in which the reactants are converted to one or more different output material(s). Chemical reactions may involve the use of catalysts, enzymes, bacteria, etc. to achieve the chemical reaction between the reactants.

The chemical production network may include one or more one or more chemical and/or mechanical process(es). The chemical production network may produce one or more output material(s) through chemical and/or mechanical processing. The chemical production network may include multiple types of production processes for producing one or more output material(s) from one or more input material(s). The chemical production network may produce one or more output material(s) from input material(s) provided to the chemical production network. The chemical production network may include a complex production network producing multiple chemical products via multiple production process(es). The chemical production network may include connected, interconnected and/or non-connected production process(es). The chemical production network may include a composite or Verbund network.

The chemical production network may include identity preserving or segregated production process(es). Identity preserving or segregated in this context may refer to environmental attribute(s) of input material(s) being preserved or segregated in the production process(es). Examples are non-fossil, e.g. renewable or recycled, input materials used to produce the one or more output material(s) without fossil content. Further examples are fossil input material(s) used to produce the one or more output material(s) with fossil content. Chemical production networks may include non-identity preserving or non-segregated production process(es). Non-identity preserving or non-segregated in this context may refer to non-fossil input material(s) being mixed with fossil input material(s) to produce the output material(s). For example, fossil and renewable input materials may be mixed to produce the output material(s) with fossil and renewable content.

The chemical production network may include one or more production process(es) with multiple production steps. The production steps included in the chemical network may be defined by the physical system boundary of the chemical production network. The system boundary may be defined by location and/or control over production processes or steps. The system boundary may be defined by a site of the chemical production network. The system boundary may be defined by production process(es) or step(s) controlled by one entity or multiple entities jointly. The system boundary may be defined by the value chain with staggered production process(es) or step(s) to the chemical end product, which may be controlled by multiple entities jointly or separately. The chemical production network may include a waste collection, a sorting step, a recycling step such as pyrolysis, a cracking step such as steam cracking, a separation step to separate intermediates of one process step and further processing steps to convert such intermediates to output material(s) leaving the system boundary of the chemical production network. The input material(s) may enter the physical system boundary of the chemical production network. The entry point(s) of the chemical production network may be marked by the entry of input material(s) to the chemical production network or the system boundary of the chemical network.

The output material(s) may leave the physical system boundary of the chemical production network. The exit point(s) of the chemical production network may be marked by the exit of output material(s) from the chemical production network or the system boundary of the chemical network.

The chemical production network may include one or more production chain(s) for the production of output materials. The production chain(s) for the production of output materials may be interconnected. The production chain(s) for the production of output materials may be interconnected with production chain(s) for the production of other output material(s). The production chain(s) for the production of output materials may include production chain(s) for the production of intermediates used to produce output materials. The production chain(s) for the production of output materials may use input material(s) provided by chemical network(s) for the production of intermediates usable to produce output materials.

One or more input material(s) may be provided to the chemical production network for producing one or more output material(s). The output material(s) may be produced from one or more input material(s) through one or more chemical process(s) of the chemical production network. The input material may comprise any input material entering the chemical production network at any entry point. The input material may include organic or inorganic input material(s). The input material may be a pre-cursor product, an intermediate material, a feedstock or a raw material used to produce one or more output material(s). The input material may be fed to the chemical production network to produce one or more output material(s). The input material may be fed to chemical production network including one or more production process(es) with multiple process steps. The input material may be fed to the chemical production network at the start of the production process or at any intermediate stage of the production process. The input materials entering the chemical production network may be used to produce one or more output material(s).

The input material may be associated with an input material identifier. The input material identifier may comprise any identifier uniquely associated with the input material. The input material identifier may be associated with the physical entity of the input material. The input material identifier may be associated with a single batch of input material. The input material identifier may be associated with a group of input materials. The identifier may be associated with multiple physical entities of the input material. The input material identifier may be associated with continuous or semi-continuous stream of input material. The input material identifier may be associated with a stream of the input material e.g. over a certain time period or from a certain supplier. The input material identifier may be linked or connected to one or more environmental attribute(s).

Environmental attribute may refer to a property related to the environmental impact. Such property may be the property of input material(s), chemical process(es), chemical production network(s) and/or output material(s). The environmental attribute may indicate an environmental performance of input material(s), chemical process(es), chemical production network(s) and/or output material(s). The environmental attribute may be derived from properties of input material(s), chemical process(es), chemical production network(s) and/or output material(s). The environmental attribute may be associated with the environmental impact of input material(s), chemical process(es), chemical production network(s) and/or output material(s) at any stage of the lifecycle of the output and/or input material(s). The stages may include providing raw material, providing feedstock, producing output materials, such as intermediate products or end products, producing discrete products by using the output materials, using output materials or discrete products, treating end-of-life products, recycling end-of-life products, disposing end-of-life products, reusing components from end-of-life products or any subset of stages. The environmental attribute may be specified or may be derived from any activity of one or more entities participating at any stage of the lifecycle of one or more material(s) or product(s).

The environmental attribute may include one or more characteristic(s) that are attributable to environmental impact of input material(s), chemical process(es), chemical production network(s) and/or output material(s). The environmental attribute may include environmental, technical, recyclability or circularity characteristics(s) associated with the environmental impact of input material(s), chemical process(es), chemical production network(s) and/or output material(s).

The environmental attribute may include one or more characteristic(s) that are attributable to the environmental impact of input material(s), chemical process(es), chemical production network(s) and/or output material(s). The environmental attribute may include environmental, technical, recyclability or circularity characteristics(s) associated with the environmental impact of input material(s), chemical process(es), chemical production network(s) and/or output material(s).

The one or more environmental attribute(s) may be attributable to the environmental impact of the output material. The one or more environmental attribute(s) may relate to environmental, technical, recyclability, circularity and/or complementary risk characteristic(s) of the output material.

Environmental characteristic(s) may specify or quantify ecological criteria associated with environmental impact. Environmental characteristic(s) may be or may be derived from measurements taken during the lifecycle. Environmental characteristics may be determined at any stage of the lifecycle and may characterize the environmental impact for such stage or up to such stage. Environmental characteristic(s) may for example include carbon footprint, greenhouse gas emissions, resource usage, air emissions, ozone depletion potential, water pollution, noise pollution, energy consumption, waste reduction, or eutrophication potential. Environmental characteristic(s) may for example include product characteristics related to the production of the product like bio based, vegetable based, animal based, halogen-free, fluorine-free, vegan, halal, kosher, palm oil-free, natural, tox-fee, volatile organic compounds-free or any combinations thereof.

Technical characteristic(s) may specify or quantify performance at least indirectly associated with the environmental impact. Technical characteristic(s) may be or may be derived from measurements taken during the lifecycle. Technical characteristics may be determined at any stage of the lifecycle and may characterize the performance for such stage or up to such stage. Technical characteristic(s) may for example include chemical composition data, raw material composition such as bio-based or recycled input material content specifying e.g. x% non fossil and y% fossil content, bill of materials, product specification data such as product purity, product form (as indication to their impact on dust formation/release), safety data, product extractability, migration data, toxicological data or ecotoxicological data, product component data, safety data, application property data, application instructions, quality data or any combinations thereof.

Circularity characteristic(s) may specify or quantify the life cycle characteristics associated with circular uses. Circularity characteristic(s) may be or may be derived from measurements taken during the lifecycle. Circularity characteristic(s) may be or may be derived from circular data recorded in one or more prior lifecycle(s) including reuse. Circularity characteristic(s) may be determined at any stage of the lifecycle and may characterize the reuse or recycling performance for such stage or up to such stage. Circularity characteristic(s) may for example include recycling data, reuse rate, recycling rate, recycling loops, reuse performance, reused quality or any combinations thereof.

Recyclability characteristic(s) may specify or quantify life cycle characteristics associated with recycling uses. Recyclability characteristic(s) may include the composition of the material including specifically tailored constituents making the material suitable for recycling. Recyclability characteristic(s) may be or may be derived from measurements taken during the lifecycle. Recyclability characteristic(s) may be or may be derived from recycling data recorded in one or more prior lifecycle(s). Recyclability characteristics may be determined at any stage of the lifecycle and may characterize the recycling performance for such stage or up to such stage. Recyclability characteristic(s) may for example include recycling data, number of reuses, recyclate composition, recyclate quality, waste stream composition, waste stream quality or any combinations thereof.

In one embodiment the output material passport or digital asset associated with the output material may include mass balanced environmental attributes related to the input material. Mass balanced environmental attributes may include environmental attributes of the input material(s) used to produce the output material, which are tracked and by mass attributable to the output material. The environmental impact of input material(s) may be determined based on input material(s) used in the chemical process(s) to produce the output material. For example, bio-based, renewable and/or recycled content of input material(s) used to produce output material may be tracked.

Examples of tracked process properties related to the environmental impact include water consumption, CO₂ emissions and/or Greenhouse Gas (GHG) emissions, amount of waste generation, mixed material generation, design for recycling, energy consumption, processing properties such as less waste or less loss of properties. The properties may be tracked based on a certificate from a certifying agency. The properties may be tracked based on inherent physical properties derived from measurements.

In one embodiment the produced output material is connected to the decentral identifier physically identifying the produced output material. The production operating apparatus may be configured to provide the decentral identifier associated with a physical entity of the produced output material. The production operating apparatus may be configured to link the decentral identifier to a physical identifier of the produced output material. The production operating apparatus may be configured to assign the decentral identifier to the physical identifier connected to the produced output material. The production operating apparatus may be configured to assign the decentral identifier to the physical identifier physically connected to the produced output material.

In one embodiment the decentral identifier relates to data associated with at least one product produced from the output material, wherein the one or more environmental attribute(s) associated with the at least one product is derived from one or more environmental attribute(s) associated with the output material. The one or more environmental attribute(s) associated with the output material may be associated with the one or more input material(s) and/or the chemical process(s) used to produce the output material. The decentral identifier may relate to any identifier uniquely associated with the output material. The decentral identifier may be associated with the physical entity of the output material. The decentral identifier may refer to a single batch of output material. The decentral identifier may be associated with a group of output materials. The identifier may refer to multiple physical entities of the output material. The decentral identifier may be associated with continuous or semi-continuous stream of output material. The identifier may refer to a stream of the output material e.g. over a certain time period or from a certain supplier.

In one embodiment the one or more environmental attribute(s) associated with the output material(s) are provided from at least one balancing account configured to store environmental attribute(s) associated with input material(s). The balancing account may relate to storage structure associated with metadata, such as an environmental attribute type. For instance, the balancing account may be associated with metadata indicating the environmental attribute type to be recycled-content or bio-based. An inbound allocator may be configured to allocate the one or more environmental attribute(s) associated with input material(s) to at least one balancing account e.g. on entry of the input material to the chemical production network. The balancing account may be associated with the respective environmental attribute type. An outbound assigner may be configured to assign at least one environmental attribute from the at least one balancing account associated with the respective environmental attribute to the decentral identifier. One or more environmental attribute(s) may be assigned to the at least one decentral identifier. Assignment may include de-allocation of the one or more environmental attributes from the balancing account associated with the respective environmental attribute type. By using the balancing accounts environmental attributes of input materials can be reliably tracked and assigned to output materials.

In one embodiment the one or more environmental attribute(s) associated with the output material(s) are provided from at least one balancing account configured to store environmental attribute(s) associated with input material(s). An inbound allocator may be configured to allocate the one or more environmental attribute(s) to at least one balancing account associated with the respective environmental attribute, e.g. on input material entering the chemical production network. The balancing account may be associated with the respective environmental attribute type. The one or more environmental attribute(s) associated with the input materials may be allocated to the balancing account associated with the respective environmental attribute type. An outbound assigner may be configured to assign or link at least one environmental attribute from the at least one balancing account associated with the respective environmental attribute type to the decentral identifier. This may include de-allocation of the one or more environmental attribute(es) from the balancing account associated with the respective environmental attribute type. By using the balancing accounts environmental attributes can be tacked through the chemical production network. This way the environmental attributes may be detached from the material flow. Attribution of environmental attribute(s) may be conducted on a mass balance basis for the chemical production network. In such approach the total mass of input materials and output materials as well as the attribution of respective environmental attribute(s) associated with input materials and output materials are balanced.

In one embodiment the one or more environmental attribute(s) are associated with at least one property related to the environmental impact of the one or more input material(s) and/or the chemical process(s). The one or more environmental attribute(s) may specify environmental properties of the input material(s) used to produce the output material and/or the one or more environmental attribute(s) may specify environmental properties of the chemical process(es) used to produce the output material. The one or more environmental attribute(s) may be generated from environmental properties of the input material(s) used to produce the output material, process data associated with the chemical processing of the input material(s) and/or energy data associated with the energy consumption of the chemical processing. The one or more environmental attribute(s) may include a recycled content associated with the input material(s) and allocated or allocatable to the output material(s), a renewable content associated with the input material(s) and allocated or allocatable to the output material(s), and/or a product carbon footprint associated with the output material(s).

In one embodiment the production operating apparatus is configured to gather environmental attributes associated with the produced output material before, during and/or after production of the output material by the chemical production network. The environmental attributes associated with the produced output material may relate to input material(s). The environmental attributes associated with input materials may be provided before, during and/or after production of the output material by the chemical production network. The environmental attributes associated with input materials may be allocated to at least one balancing account before, during and/or after production of the output material by the chemical production network. The environmental attributes associated with the produced output material may relate to environmental properties generated from process data associated with the chemical processing of the input material(s) and/or energy data associated with the energy consumption of the chemical processing. The environmental attributes associated with the produced output material may be generated before, during and/or after production of the output material by the chemical production network. The process data associated with the chemical processing of the input material(s) and/or energy data associated with the energy consumption of the chemical processing may be gathered prior, during and/or after production of the output material.

In one embodiment the output material passport or the digital asset may include the decentral identifier associated with the output material and the one or more environmental attribute(s) linked to the decentral identifier. The one or more environmental attribute(s) may be linked to the decentral identifier included in the output material passport or the digital asset. The one or more environmental attribute(s) may be stored in a data base associated with or of the output material producer for access by any output material user or consumer of the output material.

The one or more environmental attribute(s) may be stored in a data base associated with or of the output material producer for transfer to an output material user or consumer of the output material e.g. when accessed or on providing the output material. The decentral identifier may comprise any unique identifier uniquely associated with the output material producer and output material data such as the environmental attributes. The decentral identifier may include a Universally Unique IDentifier (UUID) or a Digital IDentifier (DID). The decentral identifier may be issued by a central or decentral identity issuer. The decentral identifier may be linked to authentication and/or authorization information. Via the decentral identifier and its unique association with the output material producer and output material data, such as the environmental attributes, access to the output material data may be controlled by the output material producer. This contrasts with central authority schemes, where identifiers are provided by such central authority and access to data is controlled by such central authority. Decentral in this context refers to the usage of the identifier in implementation as controlled by the data owner, such as the output material producer.

The decentral identifier may be uniquely associated with the output material or the physical entity of the output material, e.g. as packaged for transportation to the output material user or consumer of the output material. The decentral identifier may be uniquely to the one or more environmental attribute(s). The output material passport or the digital asset may include or be connected or linked to one or more digital representation(s) pointing to output material data including the environmental attribute(s) or parts thereof including the environmental attribute(s). The digital representation may comprise or be connected or linked to at least one interface to a data providing service. It may further include or be connected or linked to at least one interface to a data consuming service. It may include or be connected to or linked to an endpoint for data exchange or sharing (resource endpoint) or an endpoint for service interaction (service endpoint), that is uniquely identified via a communication protocol. The digital representation(s) pointing to output material data or parts thereof may be uniquely associated with the decentral identifier.

The output material passport or the digital asset may comprise or be connected or linked to a digital representation of output material data, such as environmental attribute(s). The digital representation may include be connected to or linked to a representation for accessing the output material data, such as environmental attribute(s) or part thereof. The digital representation may include or be connected to or linked to a representation of output material data, such as environmental attribute(s). The output material passport or the digital asset may include or be connected or linked to data related to the output material data, such as environmental attribute(s), the authentication information and the decentral identifier. The data related to the output material data, such as environmental attribute(s), may include or be connected or linked to the digital representation of the output material data, such as environmental attribute(s).

### BRIEF DESCRIPTION OF THE DRAWINGS

In the following, the present disclosure is further described with reference to the enclosed figures:
- Fig. 1: illustrates schematically an example of a chemical production network producing one or more output material(s) from one or more input material(s) in connection with a production operating system including an attribute management system.
- Fig. 2: illustrates schematically an example of attributing environmental attributes of input materials to output materials of the chemical production network.
- Fig. 3: illustrates schematically an example of attributing environmental attributes of input materials and chemical processes to an output material of the chemical production network.
- Fig. 4: illustrates schematically another example of a method or apparatus for providing environmental attributes associated with output materials to a material user as data consumer via a decentral network.
- Fig. 5: illustrates schematically an example of a method or apparatus for providing environmental attributes of output materials across value chains via the decentral network.

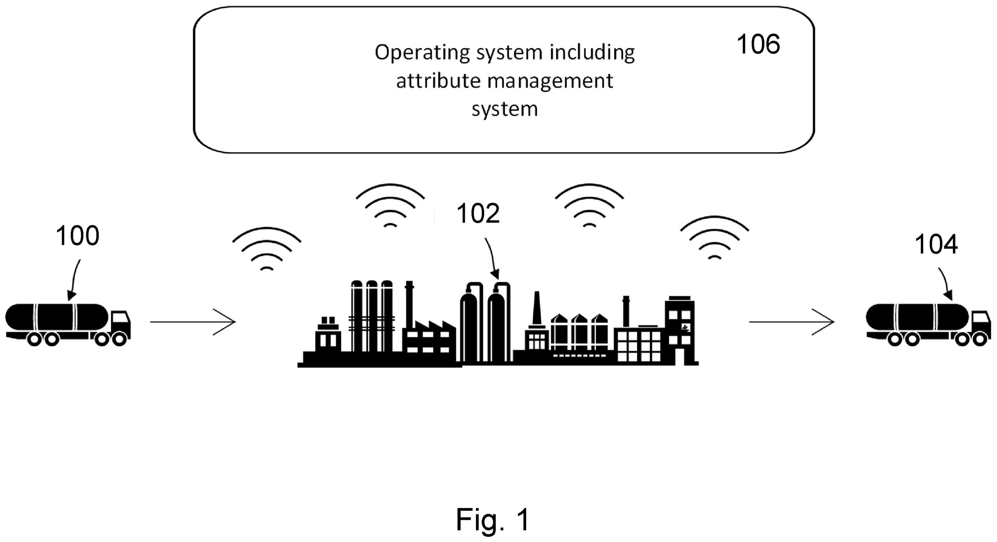

Fig. 1 illustrates an example of a chemical production network 102 producing one or more output material(s) 104 from one or more input material(s) 100 in connection with a production operating system 106 including an attribute management system.

For producing one or more output material(s) 104 different input materials 100 may be provided as physical inputs to the chemical production network 102. The physical input material(s) 100 and output material(s) 104 may be associated with one or more properties related to environmental impact. The properties related to environmental impact may be digitalized in the form of environmental attributes such as recycled or bio-based content of the input materials. The production operating system 106 may be configured to ingest such environmental attributes and to track the environmental attributes across the chemical production network 102 from input materials 100 to output materials 104.

The chemical production network 102 may include multiple interlinked processing steps. The chemical production network 102 may be an integrated chemical production network 102 with interrelated production chains. The chemical production network 102 may include multiple different production chains that have at least one intermediate product in common. The chemical production network 102 may include multiple stages of the chemical value chain. The chemical production network 102 may include the producing, refining, processing and/or purification of gas or crude oil. The chemical production network 102 may include a stream cracker, or a syngas plant connected to multiple production chains that output chemical products or materials 104 from the effluent of such plants. The chemical production network 102 may include multiple production chains that output from one or more input material(s) 100 one or more output material(s). The chemical production network 102 may include multiple tiers of a chemical value chain. The chemical production network 102 may include a physically interconnected arrangement of production sites. The production sites may be at the same location or at different locations. In the latter case the production sites may be interconnected by means of dedicated transportation systems such as pipelines, supply chain vehicles, like trucks, supply chain ships or other cargo transportation means.

The chemical production network 102 may chemically convert input materials 100 to one or more output material(s) 104. The chemical production network 102 may convert input materials 100 by way of chemical conversion to one or more output material(s) 104.

The input materials 100 may be fed to the chemical production network 102 at any entry point. The input materials 100 may be fed to the chemical production network 102 at the start of the chemical production network 102. Input materials 100 may for example make up the feedstock of a steam cracker. The input material 100 may include non-fossil input material, such as bio-based or recycled material, and/or fossil input material for the manufacture of chemical intermediates and chemical output materials 104.

The chemical production network 102 may include multiple production steps. The production steps included in the chemical production network 102 may be defined by the system boundary of the chemical production network 102. The system boundary may be defined by location or control over production processes. The system boundary may be defined by the site of the chemical production network 102. The system boundary may be defined by production processes controlled by one entity or multiple entities jointly. The system boundary may be defined by value chain with staggered production processes to an end product, which may be controlled by multiple entities separately. The chemical production network 102 may include a waste collection and sorting step, a recycling step such as pyrolysis, a cracking step such as steam cracking, a separation step to separate intermediates of one process step and further processing steps to convert such intermediates to output materials 104 leaving the system boundary of the chemical production network 102.

The production operating system 106 of the chemical production network 102 may be configured to monitor and/or control the chemical production network 102 based on operating parameters of the different processes. One process step monitored and/or controlled may be the feed of input materials 100 or the release of output materials 104. Another process step monitored and/or controlled may be the registration of environmental attributes associated with input materials 100 entering the system boundary of the chemical production network 102. Yet another process step monitored and/or controlled may be the attribution of environmental attributes to output materials 104 produced via the chemical production network 102. Yet another process step monitored and/or controlled may be the management of environmental attributes associated with input materials 100 and output materials 104 of the chemical production network 102.

The production operating system 106 may be configured to register inbound environmental attributes and to assign outbound environmental attributes. The production operating system 106 may be configured to access data related the inputs materials 100, the processes and/or the output materials 104 used in the chemical production network 102. For example, the production operating system 106 may be configured to register a recycled or bio-based content of the one or more input material(s) 100 used in the chemical production network 102 as environmental attribute. The production operating system 106 may be configured to allocate the environmental attribute to at least one balancing account associated with the recycled or bio-based content of the input materials 100. The production operating system 106 may be configured to allocate at least a part of the environmental attributes from the at least one balancing account to the at least one output material 104.

The production operating system 102 may be configured to handle environmental attributes related to the input materials 100 and output material(s) 104 of the chemical production network 102. For example, the production operating system 106 may be configured to determine environmental attributes associated with the use of input materials 100 impacting the environmental property of the chemical production network 102 and the output materials 104 produced by the chemical production network 102. Further in particular, the production operating system 102 may be configured to determine environmental attributes associated with the output materials 104. This way the production operating system 102 may be configured to store environmental attributes in balancing accounts or to withdraw environmental attributes from the balancing accounts. The environmental attributes may hence be viewed as a credit that may be deposited in an account or deducted from an account related to the input and output materials of the chemical production network 102. This way the environmental impact of the production may be tracked and/or traced.

In chemical production networks 102 multiple value chains may be linked. Additionally different input materials 100 or chemical processes impacting the environmental property of output material(s) produced by the chemical production network 102 may be used. Examples of input materials 100 impacting at least one environmental property of output materials 104 produced from such input materials 100 are recycled, renewable or bio-based input materials 104. Examples of chemical processes impacting the environmental property include chemical processes using environmentally friendly technology such as carbon capture, carbon utilization or heat pumps.

Owing to the processing of chemicals in continuous or semi-continuous production and the complexity of chemical production networks 102, traceability of the input materials through the network may be hampered. In such scenarios, an equivalent environmental attribute signifying the impact on the environmental property of output material(s) produced by the chemical production network may be allocated to balancing accounts 112 and assigned to one or more output material(s) 104 of the chemical production network 102. The environmental attributes may hence be decoupled from the physical material flow inside the chemical production network 102. Decoupling may be based on the mass balance model in that the equivalent amount assigned to the one or more output material(s) may not exceed the equivalent amount provided by input materials or processes. If an equivalent amount has been allocated to the virtual account of one environmental attribute type, it may not be allocated a second time to another virtual account of the one environmental attribute type. Environmental attribute types may be recycled, bio-based, renewable or the like. Environmental attributes may be provided in the form of digital assets or output material passports attached to the physical entity of the output material.

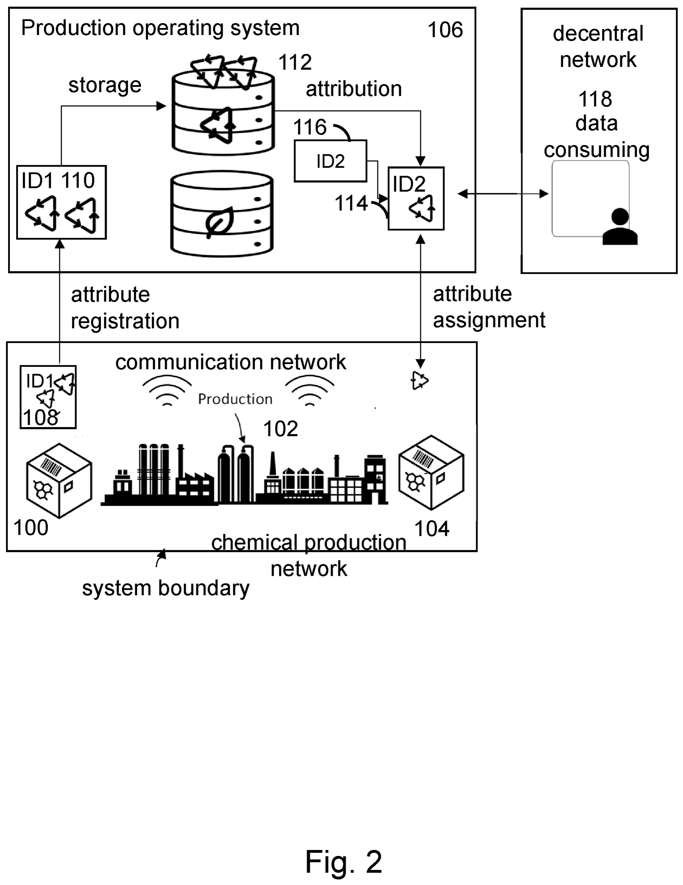

Fig. 2 illustrates schematically an example of attributing environmental attributes associated with input materials 100 to output materials 104 of the chemical production network 102.

As shown in Fig. 1 the chemical production network 102 and operations of the chemical production network 102 may be monitored and/or controlled by a production operating system 106. The production operating system 106 may be configured to track environmental attributes from input materials 100 fed to the chemical production network 102 to output materials 104 produced by the chemical production network 102. For tracking the operating system 106 may be configured to register environmental attributes associated with the input materials 100 provided to the chemical production network 102 and to attribute environmental attributes to output materials 104 produced by the chemical production network 102.

The input materials 100 such as pyrolysis oil, bio-naphtha or bio-gas may be provided to the chemical production network 102. The input materials 100 may enter the chemical production network 104 at the entry point, such as a such as a steam cracker or a syngas plant. The input materials 100 may be used in the chemical production network 102 to produce one or more output material(s) 104 from the input materials 100. Output material(s) 104 may be provided at exit points of the chemical production network 102. Further output material(s) may be MDI, TDI, PA6, EPS, PC, Polyols, Caprolactam, adipic acid, HMD, Polyamides.

On entry of the input material 100, input material data 108 may be provided via a communication network to a computing interface of the production operating system 106. A data provider, such as a QR code reader, may be configured to provide material data 108 related to the one or more input material(s) 100 and respective environmental attributes 108 to a computing interface configured to allocate the environmental attributes associated with the input materials 100. The material data 108 may include the input material identifier and environmental attributes associated with the input materials 100. The input material identifier may be associated with the physical entity of the input material 100 entering the chemical production network 102. The material data may be provided on, prior or after providing of the one or more input material(s) at entry points to the chemical production network 102.

The input material identifier may be linked to the environmental attribute(s) associated with the respective input material(s) 100, the amount of input material 100 and the certificate certifying the environmental attribute(s). The amount of input material may be a measured amount of input material 100 fed to a plant or storage of the chemical production network 102 for producing one or more output material(s) 104 from the input material(s) 100. The input material identifier associated with the respective input material 100, the environmental attribute(s) associated with the respective input material(s) 100 and the amount of input material(s) 100 provided to the chemical production network 102 may be provided to the production operating system 106.

Such data may be provided via a communication network on entry to chemical production network 102, or the data may be transferred from a computing system to the production operating system 106.

An inbound allocator 110 may be configured to allocate the one or more environmental attribute(s) to at least one balancing account 112 associated with the respective environmental attribute. For example, one balancing account 112 may relate to environmental attributes from recycled material and another balancing account 112 may relate to environmental attributes from bio-based material. The balancing account may be associated with the respective environmental attribute type, such as bio-based or recycled. Based on such association the balancing account associated with the environmental attribute type of the respective input material 100 may be selected. The environmental attributes may be allocated to the selected balancing account. For example, the account 112 for recycled material may be selected and the environmental attribute may be allocated to such account 122.

To allocate, the one or more environmental attribute(s) may be converted to balancing units and the balancing units may be allocated to the balancing account 122. The conversion may be based on a conversion factor such as mass, weight, carbon atoms, hydrogen atoms, methane equivalents or any other suitable measure for quantifying the environmental impact of the environmental attribute. The conversion factor may hence take into account the difference between producing chemical products from conventional input material(s) and producing chemical products from non-conventional input material(s) or producing chemical products from a mix of conventional and non-conventional input materials. The conversion factor may relate to differences in chemical and/or physical properties of conventional and non-conventional input material(s).

By using the balancing accounts 112 it can be ensured that environmental attributes of input materials 100 are only used once for assignment to output materials 104. This way double counting on input or output is avoided to ensure positive environmental impact can be reliable tracked and assigned to output materials 104.

An identifier provider 116 may be configured to provide the output material identifier associated with the output material produced by the chemical production network 102 and provided at the exit point from the chemical production network 102.

An outbound assigner 114 may be configured to assign at least one environmental attribute from the at least one balancing account 112 associated with the respective environmental attribute to the output material identifier ID2. One or more environmental attribute(s) may be assigned to the at least one output material identifier ID2. Assignment may include de-allocation of the one or more environmental attributes from the balancing account 112 associated with the respective environmental attribute type. Assignment may include converting one or more balancing unit(s) to one or more environmental attribute(s).

Assigning at least one environmental attribute associated with input material(s) to output material(s) may include the linking of the output materials identifier ID2 with the environmental attribute. The output material identifier ID2 may be associated with the physical entity of the output material. This way the virtual identifier of a material may be uniquely linked to the physical material. Such linking may include a physical or virtual link of identifiers uniquely associated with the physical material. For physical linking a tag or code may be physically connected to the material, e.g., by printing a QR code on the packaging. For virtual linking different identifiers associated with the physical material may be linked. For example, an order number, a batch number, LOT number or a combination thereof may be linked.

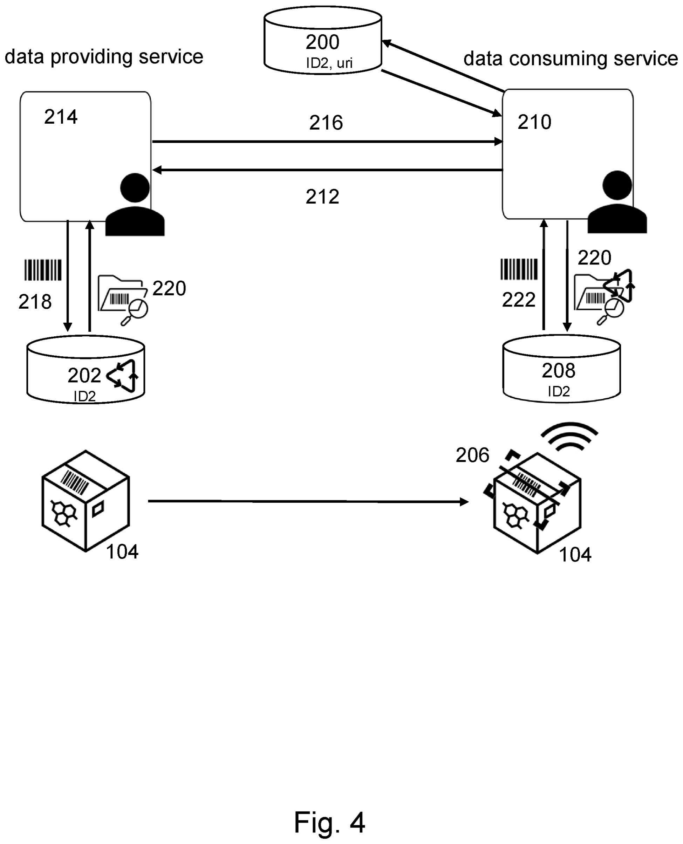

The outbound assigner 114 may be configured to provide the environmental attributes associated with the output material to a data consumer, such as a system associated with a user of the output material. The outbound assigner 114 may be configured to provide the environmental attributes associated with the output material to a decentral network as will be described in the example of Fig. 4. Environmental attributes may be provided via the above ID based schema in the form of digital assets or output material passports associated with the physical entity of the output material.

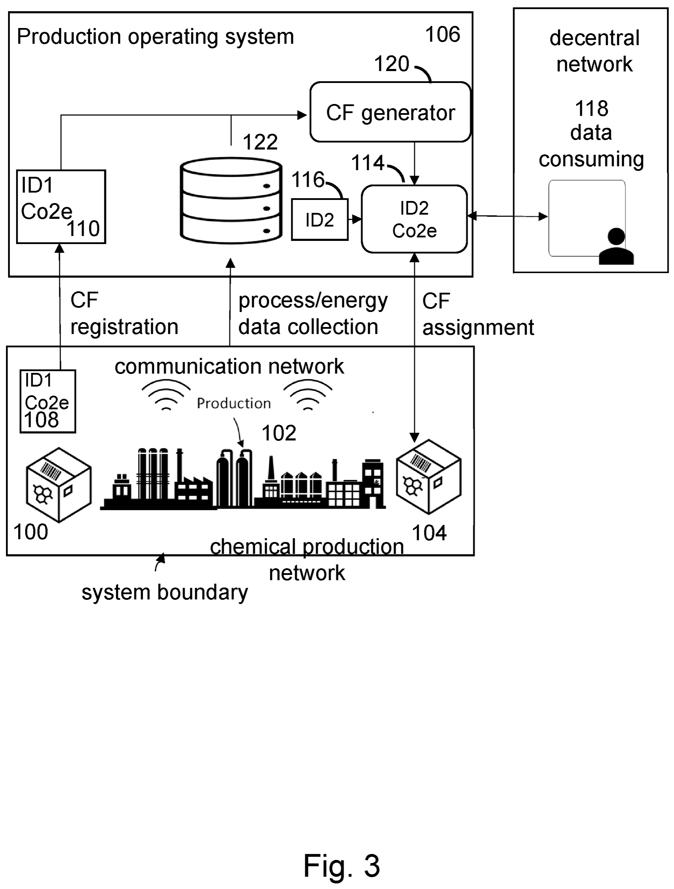

Fig. 3 illustrates schematically an example of attributing environmental attributes of input materials 100 and chemical processes to the output material 104 of the chemical production network 102.

As described in the context of Figs. 1 and 2 the chemical production network 102 and operations of the chemical production network 102 may be monitored and/or controlled by a production operating system 106. Input materials 100 such as pyrolysis oil, bio-naphtha or bio-gas may be provided to the chemical production network 102. The input materials 100 may be used in the chemical production network 102 to produce one or more output material(s) 104 from the input materials 100.

On entry of the input material 100, input material data 108 may be provided via a communication network to a computing interface of the production operating system 106. A data provider, such as a QR code reader, may be configured to provide material data 108 related to the one or more input material(s) 100 and respective environmental attributes 108 to a computing interface configured to allocate the environmental attributes associated with the input materials 100. The material data 108 may include the input material identifier and environmental attributes associated with the input materials 100. The input material identifier may be associated with the physical entity of the input material 100 entering the chemical production network 102. The input material identifier may be linked to the carbon footprint of the input material 100 as environmental attribute. The material data may be provided on, prior or after providing of the one or more input material(s) at entry points to the chemical production network 102.

The inbound allocator 110 may be configured to retrieve the one or more environmental attribute(s) and to provide such attributes to the carbon footprint (CF) generator 120. A process data provider 122 may be configured to gather process data associated with the chemical processing of the input material(s) 100 to produce the output material(s) 104. The process data provider 122 may be configured to gather energy data associated with the energy consumption of the chemical processing. The process data provider 122 may be configured to provide the process data and the energy data to the CF generator 120.

The CF generator 120 may be configured to determine the carbon footprint of the output material produced by the chemical production network. The carbon footprint of the of the output material may be determined based on the process data, the energy data and the carbon footprint of the input material(s) 100 used to produce the output material.

An identifier provider 116 may be configured to provide the output material identifier associated with the output material produced by the chemical production network 102 and provided at the exit point from the chemical production network 102.

An outbound assigner 114 may be configured to assign the determined carbon footprint to the output material identifier ID2. One or more environmental attribute(s) may be assigned to the at least one output material identifier ID2, such as described in the context of Fig. 2.

The outbound assigner 114 may be configured to provide the environmental attributes, in particular the carbon footprint, associated with the output material to a data consumer, such as a system associated with a user of the output material. The outbound assigner 114 may be configured to provide the environmental attributes associated with the output material to a decentral network as will be described in the example of Fig. 4. Environmental attributes may be provided via the above ID based schema in the form of digital assets or output material passports associated with the physical entity of the output material.

Fig. 4 illustrates schematically an example of a method or apparatus for providing environmental attributes associated with output materials to a material user as data consumer via a decentral network.

The output material 104 as produced by the chemical production network 102 may be provided in association with the digital asset as described in the context of Figs. 2 and 3. The digital asset may include the output material identifier. The digital asset may include one or more environmental attribute(s) such as the product carbon footprint, recycled content or bio-based content. The digital asset may relate to one or more environmental attribute(s) such as the product carbon footprint, recycled content or bio-based content. The digital asset may include a digital representation of one or more environmental attribute(s) such as the product carbon footprint, recycled content or bio-based content.

The digital asset may further include or relate to authentication and/or authorization information linked to the output material identifier. The authentication and/or authorization information may be provided for authentication and/or authorization of a data providing service 208 and/or data consuming service 210. The output material identifier may include or relate to a decentral identifier, that is uniquely associated with the output material. The decentral identifier may be connected to the digital representation of the environmental attributes. The digital representation may include a representation for accessing the environmental attributes or parts thereof. The decentral identifier may include a Universally Unique IDentifier (UUID) or a Digital IDentifier (DID). The decentral identifier may include any unique identifier uniquely associated with a data owner and/or output material. The data owner may be the producer of the output material. Via the decentral identifier and its unique association with the data owner and/or output material access to the material configuration data may be controlled by the data owner.

The digital asset including the digital representation of one or more environmental attribute(s) such as the product carbon footprint, recycled content or bio-based content may be stored in a decentral data base 200. The one or more environmental attribute(s) such as the product carbon footprint, recycled content or bio-based content may be stored in a data base 202 associated with the data owner, such as the producer of the output material 104.

The output material 104 may be physically delivered to a user of the output material. The output material may be connected with a QR-code having encoded the output material identifier. The user of the output material may read the QR-code through a QR-code reader 206. The output material identifier may be provided to a data base 208 associated with the user or consumer of the output material 104. In other embodiments the user or consumer of the output material may retrieve the output material identifier through the decentral data base 200.

The data owner in this example may be the input material producer, the output material producer, the output material user, the end product producer. The data owner may comprise any entity generating data. The data generating node may be coupled to the data owner or the entity owning or producing physical products from or for which data is generated. The data may be generated by a third-party entity on behalf of the entity owning physical products from or for which data is generated.

The data consuming service 210 may comprise computer-executable instructions for accessing and/or processing data, such as output material data, associated with the data owner. The data providing service 210 may comprise computer-executable instructions for providing and/or processing data, such as plastic additive data, associated with the data owner for accessing and/or processing by the data consuming service 214.

Based on the received output material identifier a request to access the environmental attributes associated with the output material identifier may be triggered by the data consuming service 210 as signified by arrow 212. The output material identifier may be provided to the data providing service 214 associated with or of the producer of the output material 104. In addition, authentication and/or authorization information may be provided.

The request may be authenticated and/or authorized to access the environmental attributes associated with the output material identifier. Based on successful authorization and/or authentication access to the environmental attributes associated with the output material identifier may be granted.

For access the output material identifier may be provided to the data providing service 214 as signified by arrow 212. The data providing service 214 may use the received output material identifier to retrieve the environmental attributes associated with the output material 104 as signified by arrows 218 and 220. The environmental attributes associated with the output material 104 provided to the data providing service 214 may be provided to the data consuming service 210 as signified by arrow 216. The environmental attributes associated with the output material 104 may be stored in the data base 208 associated with the user of the output material 104 as signified by arrow 220.

Through the output identifier or decentral identifier the environmental attributes can be uniquely associated with the output material. Through the decentral network the environmental attributes may be transferred between the producer of the output material and the user of the output material. This way the environmental attributes can be shared with unique association to the output material and without central intermediary directly between the value chain players. This allows for transparency of environmental attributes across the value chain and positive environmental impacts from output materials produced by the chemical production network 102 can be tracked through the value chain.

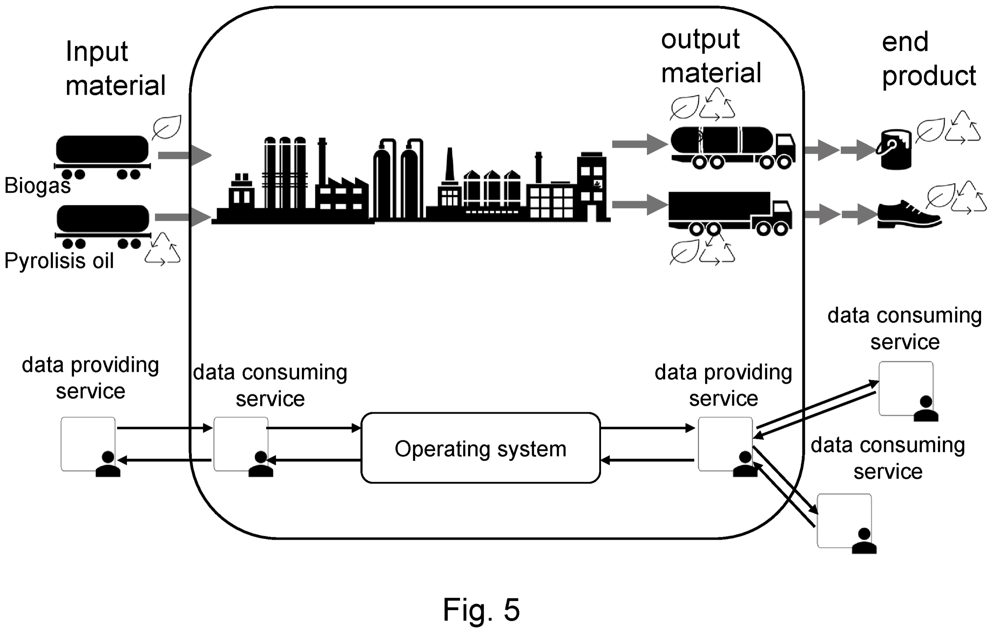

Fig. 5 illustrates schematically an example of a method or apparatus for providing environmental attributes associated with output materials across value chains via the decentral network.

In the example of Fig. 5 a fully connected value chain including the chemical production network is illustrated. In the example, the input material provider, the output material producer, the output material user and the end product producer may be connected to the decentral network as described in the context of Fig. 4. Environmental attributes may be provided via the ID based schema described in the context of Figs. 2-4 in the form of digital assets or output material passports associated with the physical entity of the input material, the output material, any intermediate product or the end product.

The input material provider may provide the input materials such as bio-gas or pyrolysis oil. The environmental attributes of the input material may be provided through the data providing service connected to the decentral network as described in the context of Fig. 4. The output material producer may produce the output material from the input material(s) provided to the chemical production network. The output material producer may access the environmental attributes associated with the input material through a data consuming service connected to the decentral network as described in the context of Fig. 4. The output material producer may manage the environmental attributes via the production operating system as described in the context of Figs. 1 to 3. The output material producer may assign the environmental attributes associated with the input materials or environmental attributes associated with the chemical production network such as the carbon footprint, to the output materials as described in the context of Figs. 1 to 3.

The output material producer may provide the environmental attributes associated with the output material through the data providing service connected to the decentral network as described in the context of Fig. 4. The output material user or the end product producer may access the environmental attributes associated with the output material through the data consuming service connected to the decentral network as described in the context of Fig. 4.

The respective data owners in this example may be the input material producer, the output material producer, the output material user, the end product producer. The data owner may comprise any entity generating data. The data generating node may be coupled to the data owner or the entity owning or producing physical products from or for which data is generated. The data may be generated by a third-party entity on behalf of the entity owning physical products from or for which data is generated.

The data consuming service may comprise computer-executable instructions for accessing and/or processing data, such as chemical product data, associated with the data owner. The data providing service may comprise computer-executable instructions for providing and/or processing data, such as plastic additive data, associated with the data owner for accessing and/or processing by the data consuming service.

In the example of Fig. 5 the decentral identifier may relate to the end product. Such decentral identifier may be provided to the value chain participants. Via the end product specific decentral identifier data associated with the end product produced from the output material may be gathered across the production chain and assigned to the end product specific decentral identifier. For example, the one or more environmental attribute(s) associated with the end product may be derived from the environmental attribute(s) associated with the output material, the input material or any other product entity present in the value chain of the end product.

This way the environmental attributes of input materials, output materials and any products produced from output materials may be tracked through the value chain up to the end product. By tracking the environmental attributes of materials in such way the information can be made transparent across the value chain while the information flow can be controlled by the participants in the supply chain. In addition, the environmental attributes can be handled according to the individual participants needs by production operating systems as described in the context of Figs. 1 to 3. Overall, such tracking enables tracking of positive environmental impact by individual supply chain participants, which makes positive environmental impacts transparent and attributable to individual supply chain participants.

The present disclosure has been described in conjunction with preferred embodiments and examples as well. However, other variations can be understood and effected by those persons skilled in the art and practicing the claimed invention, from the studies of the drawings, this disclosure and the claims.

Any steps presented herein can be performed in any order. The methods disclosed herein are not limited to a specific order of these steps. It is also not required that the different steps are performed at a certain place or in a certain computing node of a distributed system, i.e. each of the steps may be performed at different computing nodes using different equipment/data processing.

As used herein "determining" also includes "initiating or causing to determine", "generating" also includes "initiating and/or causing to generate" and "providing" also includes "initiating or causing to determine, generate, select, send and/or receive". "Initiating or causing to perform an action" includes any processing signal that triggers a computing node or device to perform the respective action.

In the claims as well as in the description the word "comprising" does not exclude other elements or steps and the indefinite article "a" or "an" does not exclude a plurality. A single element or other unit may fulfill the functions of several entities or items recited in the claims. The mere fact that certain measures are recited in the mutual different dependent claims does not indicate that a combination of these measures cannot be used in an advantageous implementation.

Any disclosure and embodiments described herein relate to the methods, the systems, devices, the computer program element lined out above and vice versa. Advantageously, the benefits provided by any of the embodiments and examples equally apply to all other embodiments and examples and vice versa.

All terms and definitions used herein are understood broadly and have their general meaning.

## Claims

1. A system for producing an output material associated with a digital asset, the system comprising:
- a chemical production network configured to produce an output material, wherein the output material is produced from one or more input material(s) through one or more chemical process(s) of the chemical production network, wherein the one or more input material(s) are associated with one or more environmental attribute(s);
- a production operating apparatus configured to generate the digital asset by providing a decentral identifier associated with the produced output material and linking the decentral identifier to the one or more environmental attribute(s) associated with the one or more input material(s),
wherein the one or more environmental attribute(s) associated with the output material are provided from at least one balancing account configured to store environmental attribute(s) associated with the one or more input material(s),
wherein linking the decentral identifier to the one or more environmental attribute(s) includes de-allocation of the one or more environmental attribute(s) from the at least one balancing account.

2. The system of claim 1, wherein the produced output material is connected to the decentral identifier physically identifying the produced output material.

3. The system of any of the preceding claims, wherein the one or more environmental attribute(s) are allocated to the at least one balancing account on the one or more input material(s) entering the chemical production network.

4. The system of any one of the preceding claims, wherein the at least one balancing account relate to a storage structure associated with one or more environmental attribute type(s), in particular wherein the at least one balancing account is selected based on the environmental attribute type(s) of the at least one balancing account and the environmental attribute type of the input material(s) and the environmental attribute(s) are allocated to the selected at least one balancing account.

5. The system of any of the preceding claims, wherein the one or more environmental attribute(s) are associated with at least one property related to the environmental impact of the one or more input material(s).

6. The system of any of the preceding claims, wherein the one or more environmental attribute(s) include a recycled content associated with the input material(s), a renewable content associated with the input material(s) or a bio-based content of the input material(s).

7. The system of any of the preceding claims, wherein the production operating apparatus is configured to allocate the one or more environmental attribute(s) to the at least one balancing account based on input material data related to the one or more input material(s), wherein the input material data includes input material identifier(s) associated with the one or more input materials and the one or more environmental attribute(s).

8. The system of any of the preceding claims, wherein linking the decentral identifier to the one or more environmental attribute(s) associated with the one or more input material(s) includes converting one or more balancing unit(s) stored in the at least one balancing account to the one or more environmental attribute(s).

9. The system of any of the preceding claims, wherein the one or more environmental attribute(s) are converted to balancing units and the balancing units are allocated to the at least one balancing account, in particular wherein the conversion is based on a conversion factor relating to differences in chemical and/or physical properties of conventional and non-conventional input material(s).

10. The system of any of the preceding claims, wherein the environmental attribute(s) associated with input material(s) are allocated to the at least one balancing account before, during and/or after production of the output material by the chemical production network.

11. The system of any of the preceding claims, wherein the one or more environmental attribute(s) are decoupled from a physical material flow inside the chemical production network by allocation to the at least one balancing account and assignment to the output material.

12. The system of any of the preceding claims, wherein the production operating system is configured to store the one or more environmental attribute(s) in the at least one balancing account and to de-allocate the one or more environmental attribute(s) from the at least one balancing account.

13. A method for producing an output material associated with a digital asset, wherein the method comprises:
- producing the output material from one or more input material(s) through one or more chemical process(s) of a chemical production network, wherein the one or more input material(s) are associated with one or more environmental attribute(s);
- generating the digital asset by
providing a decentral identifier associated with the produced output material and the one or more environmental attribute(s) associated with the one or more input material(s);
linking the decentral identifier and the one or more environmental attribute(s);
- providing the produced output material in association with the digital asset
wherein the one or more environmental attribute(s) associated with the output material(s) are provided from at least one balancing account configured to store environmental attribute(s) associated with input material(s),
wherein linking the decentral identifier to the one or more environmental attribute(s) includes de-allocation of the one or more environmental attribute(s) from the at least one balancing account.

14. An output material associated with a digital asset, wherein the output product is produced and the digital asset is generated by the system according to any of claims 1 to 12 or according to the method of claim 13.

15. A computer-implemented method for generating a digital asset associated with an output material, wherein the output material is produced from one or more input material(s) through one or more chemical process(s) of a chemical production network, wherein the one or more input material(s) and/or the one or more chemical process(s) are associated with environmental attribute(s), the method comprising:
- providing a decentral identifier associated with the produced output material and one or more environmental attribute(s) associated with the one or more input material(s) used to produce the output material;
- linking the decentral identifier and the one or more environmental attribute(s), wherein the one or more environmental attribute(s) associated with the output material(s) are provided from at least one balancing account configured to store environmental attribute(s) associated with input material(s), wherein linking the decentral identifier to the one or more environmental attribute(s) includes de-allocation of the one or more environmental attribute(s) from the at least one balancing account;
- providing the digital asset in association with the produced output material, wherein the one or more environmental attribute(s) associated with the output material is made accessible to a user of the output material through the digital asset.
